# EUROPEAN PATENT APPLICATION

(11) **EP 4 176 924 A1**
(43) Date of publication of application: **10.05.2023**
(21) Application number: 21207084.1
(22) Date of filing: 09.11.2021
(51) Int. Cl.: A61N 1/375, A61N 1/372

(54) **IMPLANTABLE MEDICAL DEVICE AND FEEDTHROUGH ASSEMBLY THEREFOR**

(71) Applicant: BIOTRONIK SE & Co. KG, 12359 Berlin (DE)
(72) Inventor: Henschel, Martin, 13125 Berlin (DE)
(74) Representative: Biotronik Corporate Services SE

(57) **Abstract**

The invention refers to a feedthrough assembly (10) for an implantable medical device which is less cost-intensive during manufacturing and more suitable for smaller medical devices, wherein the feedthrough assembly comprises an electrically conducting flange (11) having a distal face (lla) and a proximal face (11b), wherein the flange (11) accommodates a feedthrough pin (14) hermetically sealed within a through-hole (11.4) of the flange (11) with an electrode tip (15) located at the distal end of the feedthrough pin (14), wherein the feedthrough pin (14) is electrically insulated with regard to the flange (11), wherein the flange (11) comprises at least one electrically conducting ground terminal block (16) comprising solderable material, wherein each of the at least one ground terminal block (16) is friction-locking and/or positive-locking fixed to the flange (11) at its proximal face (11b), thereby establishing an electrical connection between the flange (11) and the at least one ground terminal block (16). The invention further refers to a respective implantable medical device and manufacturing methods for such feedthrough assembly and implantable medical device, respectively.

## Description

The present invention refers to an implantable medical device, such as a pacemaker or a defibrillator device, and a feedthrough assembly therefor as well as a manufacturing method for such feedthrough assembly and for such implantable medical device.

Implantable medical devices can be active or passive. An active medical device provides a pre-defined therapeutic effect to a patient whereas a passive device is usually focused on measuring at least one bodily parameter of the patient. An active medical device may comprise passive functionality, as well. An example of an active medical device is an implantable intracardiac pacemaker (implantable leadless pacemaker, ILP). Such pacemaker is entirely implanted into a heart's ventricle or atrium and applies electrical stimulation in the form of pulses to the heart in order to generate a physiologically appropriate heartrate and/or in the form of shocks for cardioversion or defibrillation in order to restore a more normal heart rhythm. Alternative or additional functions of an intracardiac device comprises providing of other electrical or electromagnetic signals to the heart or its surrounding tissue, sensing electrical or electromagnetic signals or other physiological parameters of the heart and/or its surrounding tissue.

An implantable intracardiac pacemaker or other medical device of reduced size and component number typically comprises a hermetically sealed housing. For an electrical connection of one contact, e.g. in form of an electrode, accessible from outside of the housing to an electric circuitry located inside the housing often a feedthrough assembly is used. The feedthrough assembly is fixed, for example, by welding to the housing such that the housing is hermetically sealed. In other words, the feedthrough assembly forms a part of the housing and often consists of - at least partly - biocompatible material. The feedthrough assembly is set up to provide a seat for the electrode and an electrical isolation of it with regard to the housing and other parts of the feedthrough assembly. In many cases the feedthrough assembly further provides the ground connection of the circuitry, for example by forming an electric connection to the electrically conductive housing. Accordingly, there is the need to electrically connect a feedthrough component that is electrically coupled with the housing to an electric module located inside the housing. Feedthrough biocompatible components usually do not consist of solderable material whereas typically an electrically conducting connection to an electric module/circuitry is provided by surface mount technology (SMT).

Today's known solutions to fix a solderable component to a non-solderable component are sophisticated welding or hard soldering methods. For example, a hard solderable pin material is brazed with a gold braze into the biocompatible feedthrough component. However, these pin materials are often not solderable using soft solder technologies, such as reflow soldering, but instead act as a bridging material. An additional terminal block out of a soft solderable material is therefore laser-welded or hard soldered to that bridging pin material. The terminal block surface needs to be pre-tinned or coated to prevent it from oxidation. Welding the terminal block material directly to the feedthrough component by means of laser-welding creates cracks in the intermetallic phase. Even by using the method of resistance welding, where only a small pool of intermetallic phase is created and the mixing of the materials is reduced in comparison to laser welding, cracks can be reduced but not completely excluded.

To connect a biocompatible feedthrough component to an electronic module thus requires sophisticated welding or brazing equipment. Brazing equipment is highly cost intensive because of the need of high temperatures and a vacuum environment under which the brazing process needs to take place. The process time is also very long for a batch of components. Additionally, it is very challenging to align the small components of the implantable device and keep them in place. This may further cause yield loss.

Further, the above solutions are difficult to automate and are not suitable for smaller medical devices and/or feedthrough assemblies.

Accordingly, it is an objective of the present invention to provide an implantable medical device, a respective feedthrough and corresponding manufacturing methods, which are less cost-intensive and more suitable for smaller medical devices.

The above problem is solved by a feedthrough assembly for an implantable medical device with the features of claim 1, an implantable medical device with the features of claim 9, a manufacturing method for a feedthrough assembly with the features of claim 10 and a manufacturing method for an implantable medical device with the features of claim 13.

In particular, the above problem is solved by a feedthrough assembly for an implantable medical device, the feedthrough assembly comprising a, for example substantially disc-shaped, electrically conducting flange having a distal face and a proximal face, wherein the flange accommodates a feedthrough pin hermetically sealed within a through-hole of the flange with an electrode tip located at the distal end of the feedthrough pin, wherein the feedthrough pin is electrically insulated with regard to the flange, wherein the flange comprises at least one electrically conducting ground terminal block, comprising solderable material, wherein each of the at least one ground terminal blocks is friction-lockingly and/or positive-lockingly fixed to the flange at its proximal face, thereby establishing an electrical connection between the flange and the at least one ground terminal block.

The implantable medical device may be an implantable intracardiac pacemaker (implantable leadless pacemaker, ILP). that is configured to apply electrical stimulation in the form of pulses to the heart in order to affect the heartrate in such a way that a normal heart rhythm is restored. It may also be configured to apply electrical shocks for cardioversion or defibrillation. Alternative or additional functions may be providing other electrical or electromagnetic signals to the heart or its surrounding tissue, sensing electrical or electromagnetic signals or other physiological parameters of the heart and/or its surrounding tissue. The implantable medical device may contain any combination of the above functions. The implantable medical device may be further a implantable cardiac monitor, a implantable neuro stimulator, implantable pacemaker, implantable defibrillator or any kind of implantable sensor like a pressure sensor.

The inventive feedthrough assembly is suitable for such implantable medical device which usually comprises a housing, wherein the feedthrough assembly is fixed to the housing such that the housing is hermetically sealed by the feedthrough assembly. The at least one ground terminal block is friction-lockingly and/or positive-lockingly fixed to the feedthrough flange and configured to be electrically connected to a respective terminal of an electrical circuitry by soldering, wherein the electrical circuitry is located within the housing. The feedthrough assembly may be welded to the implant housing to guarantee its hermetical sealing.

The above feedthrough assembly and implantable medical device provide a simple and long term stable electrical and mechanical connection of the ground terminal block to the feedthrough assembly and thereby to the housing of the medical device. As the ground terminal block comprises solderable material, it is possible to solder one end of the ground terminal block to a respective terminal of the electrical circuitry (e.g. an electronic circuit board) located within the housing. Due to the mechanical connection of the ground terminal block by a friction-locking and/or positive-locking fixation the material of the feedthrough flange may be structurally and chemically different from the material of the ground terminal block. The manufacturing method described below may easily be to implement in an automated manufacturing process which needs only low invest sums for welding and/or assembly equipment. The design described in this disclosure reduces process complexity for a cost effective and safe product. Further, it opens a new world for application of a variety of solderable materials to ensure a robust and perfectly adjusted SMT-reflow process for the connection to the electrical circuitry located inside the housing. Accordingly, it enables surface mount technology for an easy to assemble feedthrough assembly.

The electrically conducting flange of the feedthrough assembly provides the hermetical closure of the housing and provides an electrical connection to the housing at the same time. As the flange quasi forms a part of the housing, it may comprise a biocompatible material at least at its distal face. The proximal face of the flange is opposite the distal face and is at least partly located within the housing after assembly of the implantable medical device. In one embodiment, the flange consists of biocompatible, electrically conducting material comprising at least one material of the comprising Titanium, a Titanium alloy such as Nitinol, stainless steel such as MP35N, Platinum or a Platinum alloy such as a platinum-iridium alloy. The material of the flange may be chosen such that it is attachable to the housing of the medical device thereby forming a hermetically tight seal. The flange may have a circular or angular cross section and is, for example, in its form adapted to the form of the distal end of the housing of the medical device. In one embodiment, the flange forms a circumferential recess at its proximal face adapted to the distal end of the housing and to attach the housing to the flange, e.g. by welding.

The electrically conducting feedthrough pin and the electrically conducting electrode tip at its distal end provide an electrical connection from the outside of the medical device to the electrical circuitry located inside the housing for transmitting electrical signals, e.g. electrical signals from the patient's heart, i.e. from the patient's tissue to the medical device or from the medical device to the patient's tissue, e.g. sensing signals and/or providing pacing or defibrillation signals. The feedthrough pin and the electrode tip are electrically insulated from the flange and run along a, for example, cylindrical through-hole of the flange. However, the feedthrough pin is fixed within the through-hole of the flange fully surrounded by an electrically insulating material, for example a ceramic (e.g. Al₂O₃) or glass-to-metal-seal (GTMS), that is located between the feedthrough pin and inner wall of the through-hole, thereby enclosing the feedthrough pin and forming the hermetic seal. The feedthrough pin may have a round or angular cross section. The electrode tip may be attached to the feedthrough pin by welding. Alternatively the electrode tip and the feedthrough pin may be formed as one piece. The electrode tip may be formed for reliably connecting to a standard pacing electrode connecting module (e.g. for a standard pacing electrode IS-1 or IS-4). The feedthrough pin may be soldered in any suitable way to a respective terminal of the electrical circuitry located within the housing of the medical device.The electrode tip may comprise a fractal coating to enhance the conductive surface.

The feedthrough pin may comprise at least one material of the group comprising platinum, platinum-iridium alloy, neobium, niobium alloy, titanium, titanium alloy, stainless steel (in particular MP35N). and the electrode tip may comprise at least one material of the same group

The at least one ground terminal block provides the ground connection of the electrical circuitry and forms an electrical connection via the flange to the housing of the medical device. It may have a cylindrical shape, a cuboid shape or a disc-shape and is fixed to the flange by friction-locking and/or positive-locking. The electrical connection is provided by a direct contact of at least one front face and/or at least one side face (e.g. shell surface) to a ground surface or inner surface of a respective recess. The feedthrough assembly may have at least two or at least three ground terminal blocks. The mechanical connection to the flange allows to use a hard or soft solderable material for the direct connection with the respective terminal of the electrical circuitry. For exact soldering with the respective terminal of the electrical circuitry each ground terminal block may comprise a protruding solder pin at one face, e.g. its proximal face, which comprises solderable material and/or a coating of solderable material. Alternatively the ground terminal block(s) may comprise a flat proximal face, whereby proximal face of the terminal block is preferably at least slightly distanced from the flange. In this embodiment the terminal block(s) may be pin-shape or cylindrical.

In one embodiment, each of the at least one ground terminal blocks is friction-locking and/or positive-locking fixed, e.g. by press fitting, within one of at least one recesses located at the proximal face of the flange. If the at least one ground terminal block is fixed within one recess, it is protected within this recess. Alternatively, the at least one ground terminal block may comprise a recess which is friction-locking and/or positive locking fixed to a respective protrusion extending from the proximal face of the feedthrough flange.

In one embodiment the outer diameter of the at least one ground terminal block is slightly, for example by 1% to 5%, greater than the inner diameter of the respective recess in order to ensure reliable mechanical fixation of the ground terminal block within this recess.

In one embodiment, the at least one recess comprises a chamfer at the proximal end of its inner side surface that provides the positive-locking connection thereby facilitating the insertion of the at least one ground terminal block into the respective recess. Similarly, the ground terminal block may comprise a chamfer at a distal and/or proximal end of its side face or shell surface or the outer rim of its distal face.

In one embodiment, the at least one terminal block may comprise at least one material of the group comprising Copper/Iron/Aluminum alloys (for example with an electroless/electrolytic Gold finish, with an electroless nickel immersion gold (ENIG) finish, and/or with an electroless nickel electroless palladium immersion gold (ENEPIG) / electroless nickel immersion palladium immersion gold (ENIPIG) finish (electroless Palladium) finish), Nickel alloys (for example with a Gold coating, a Palladium coating and/or a solder dip), Copper alloys (for example with a solder protectant), Copper/Nickel alloys (for example with CuNi44/CuNi45 (for example with ENIG coating)), Beryllium/Copper alloys (for example without coating or with an ENIG or Palladium coating), Phosphor/Bronze alloys (for example without coating or with Pb510 coating).

In one embodiment, the at least one ground terminal block comprises a material that is softer than the material of the feedthrough flange. This means that the hardness of the material of the flange is greater than the hardness of the at least one terminal block. Caused by the difference in hardness, the flange material may deform the material of the at least one ground terminal block during fixation of the at least one ground terminal block to the flange, for example within a respective recess. The hardness of the materials may be measured using a known indentation hardness test (e.g. Vickers hardness test).

In one embodiment, the at least one recess of the flange may comprise at least one protrusion at an inner rim of the recess adapted to provide the friction-locking and/or positive-locking fixation of the respective ground terminal block, for example by notching or scratching into the surface of the respective ground terminal block.

In one embodiment, the at least one ground terminal block is press-fitted into or locked in place using a bayonet lock or a threaded lock at the corresponding recess at the proximal face of the flange. This is an alternative possibility to provide the friction-locking and/or positive locking connection of the ground terminal and the flange.

The above object is further solved by a manufacturing method for a feedthrough assembly as described above, wherein the method comprises the following steps:
- Providing the flange,
- Fixing the feedthrough pin hermetically sealed and electrically insulated with regard to the flange within the though-hole of the flange, for example as indicated above,
- optional attachment of the electrode tip to the distal end of the feedthrough pin, for example by welding, as indicated above,
- Friction-locking and/or positive-locking fixing of at least one ground terminal block at the proximal face of the flange, thereby establishing an electrical connection between the flange and the at least one ground terminal block.

The feedthrough pin and the electrode tip may be formed as one piece. If feedthrough pin and electrode tip are not formed as one piece, the electrode tip may be optionally attached to the distal end of the feedthrough pin.

Some embodiments with regard to friction-locking and/or positive-locking fixing of the ground terminal block to the flange are described above.

In one embodiment, the at least one ground terminal block is solder-bumped, preferably pre-tinned, prior or post fixing at the flange in order to provide a reliable solder connection to the respective terminal of the electric circuitry.

The above object is further solved by a manufacturing method for an implantable medical device as described above, wherein the method comprises the following steps:
- Manufacturing the feedthrough assembly according to the method described above,
- Fixing and electrically connecting the at least one ground terminal block to a respective terminal of an electrical circuitry by soldering, for example by using an automated reflow process,
- Inserting the electrical circuitry within the housing and afterwards welding the feedthrough assembly to the implant housing, thereby closing the housing hermetically.

The electrical circuitry within the housing may comprise at least one electronic circuit board comprising electric components such as a processor, a voltage transformer, a signal generator, a sensor for electromagnetic signals, a battery or other power source, or a high voltage capacitor. The electrical circuitry comprises at least one terminal for connection to the feedthrough pin and one terminal for connection to each of the at least one ground terminal block.

The above explanations to the embodiments of the feedthrough assembly and the medical device apply to the above methods, as well. It is therefore referred to the above explanations.

The above described feedthrough assembly, medical device allows a combination of a solderable material of the at least one ground terminal block with a biocompatible Titanium or similar material flange, irrespective of the solderability or weldability of these materials. The solderable material of the at least one terminal block reduces the risk of crack formation thereby ensuring a reliable medical device over its lifetime. A possible coating of the at least one ground terminal block does not need to be heat resistant to the temperatures needed for the gold brazing process or GTMS process to create a leak tight feedthrough - the ground terminal blocks are easy to assembly afterwards. It is now possible to use any suitable coating material which improves solderability for a standard and reliable SMT-process. Further, it is easy to assemble the at least one ground terminal to the flange, for example by using a uniaxial press. Further, it is possible to assemble at least two ground terminal blocks to one flange at once thereby saving process time. Further, the above method and device save high investment costs for welding equipment and high temperature brazing vacuum ovens as well as for sophisticated brazing fixtures made from a high temperature resistant material.

The present invention will now be described in further detail with reference to the accompanying schematic drawings, wherein
- Fig. 1: shows a distal end of an embodiment of an inventive implantable medical device with an inventive feedthrough assembly in a longitudinal-sectional view,
- Fig. 2: depicts the embodiment of the feedthrough assembly of Fig. 1 in a longitudinal-sectional view,
- Fig. 3: shows a first manufacturing step of the feedthrough assembly of Fig. 2 in a longitudinal-sectional view,
- Fig. 4: shows the manufacturing step of Fig. 3 in a perspective bottom view,
- Fig. 5: depicts the feedthrough assembly of Fig. 2 in a bottom view,
- Fig. 6: depicts a first enlarged section of Fig. 5,
- Fig. 7: shows a ground terminal block of the feedthrough assembly of Fig. 2 in a bottom view prior assembly,
- Fig. 8: outlines a second manufacturing step of the feedthrough assembly of Fig. 2 in a longitudinal-sectional view,
- Fig. 9: depicts a second enlarged section of Fig. 5,
- Fig. 10: depicts the embodiment of a medical device of Fig. 1 as a functional diagram.

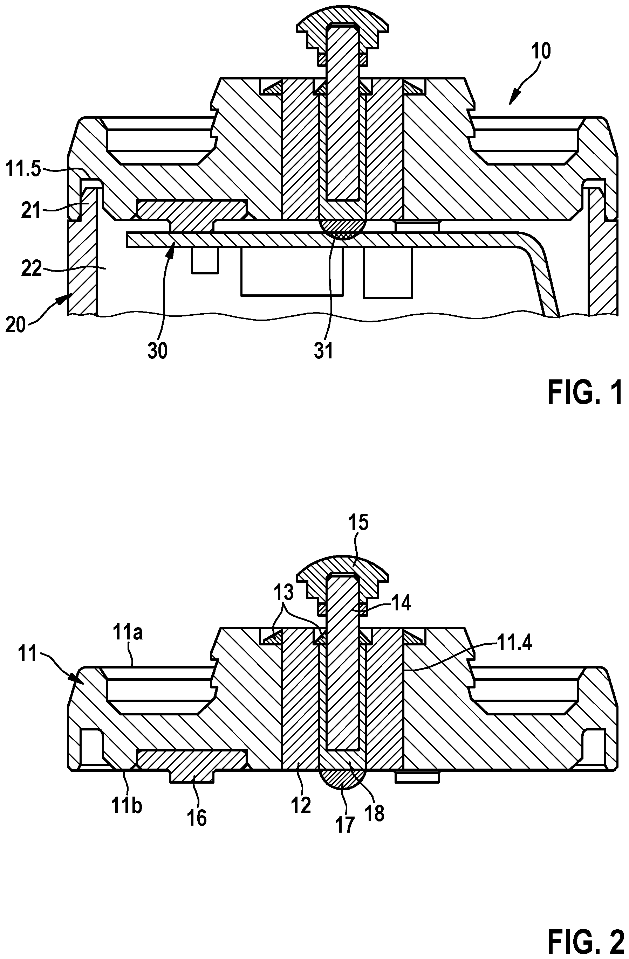

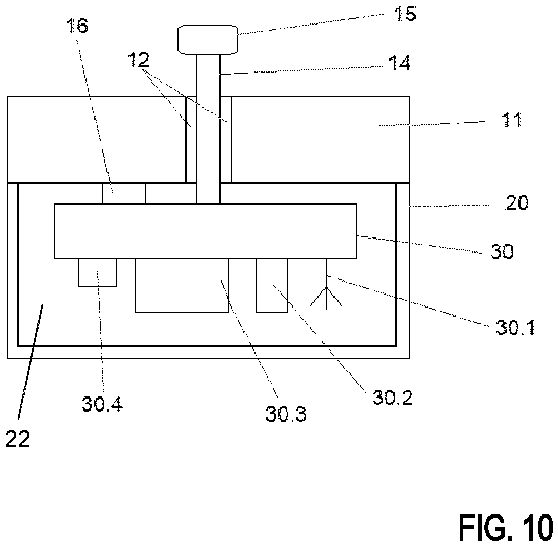

Fig. 1 and 10 illustrate an implantable medical device, e.g. an ILP, comprising a feedthrough assembly 10, a housing 20 and an electric circuitry in form of an electronic circuit board 30. The feedthrough assembly 10 is fixed to the distal end 21 of the housing 20, for example by welding such that the housing 20 is hermetically sealed by the feedthrough assembly 10 thereby forming an inner volume 21 of the housing 20 that contains the electronic circuit board 30.

The feedthrough assembly 10 as depicted in Fig. 2 to 9 comprises a generally disc-shaped and electrically conducting flange 11 having a distal face 11a and a proximal face 11b. A pin-shaped electrode consisting of a feedthrough pin 14 and an electrode tip 15 at its distal end, is accommodated within a through-hole 11.4 of the flange 11 where it is electrically insulated from the flange 11 by a hollow cylindrical insulator 12. The insulator 12 consists of an electrically insulating material such as ceramic, for example Al₂O₃, or a glass-to-metal-seal. At the distal face 11a of the flange 11, the feedthrough pin 14 is further hermetically sealed within the insulator 12 using a gold braze or glass-to-metal-seal 13. The electrical connection of the feedthrough pin to a respective terminal of the electronic circuit board 30 is further provided by platinum paste 18 that is accommodated at the proximal end of the feedthrough pin 14 within the through-hole of the insulator 12. Additionally, a pre-tinning 17 of the platinum paste 18 may be provided. Alternatively, the feedthrough pin 14 may be provided with gold braze or GTMS for good solderability to a respective terminal at the electronic circuit board 30 as shown at a location denoted with reference sign 31 in Fig. 1. Thereby an electrical connection of the electrode tip 15 to the electric circuitry at the electronic circuit board 30 via the feedthrough pin 14 is provided.

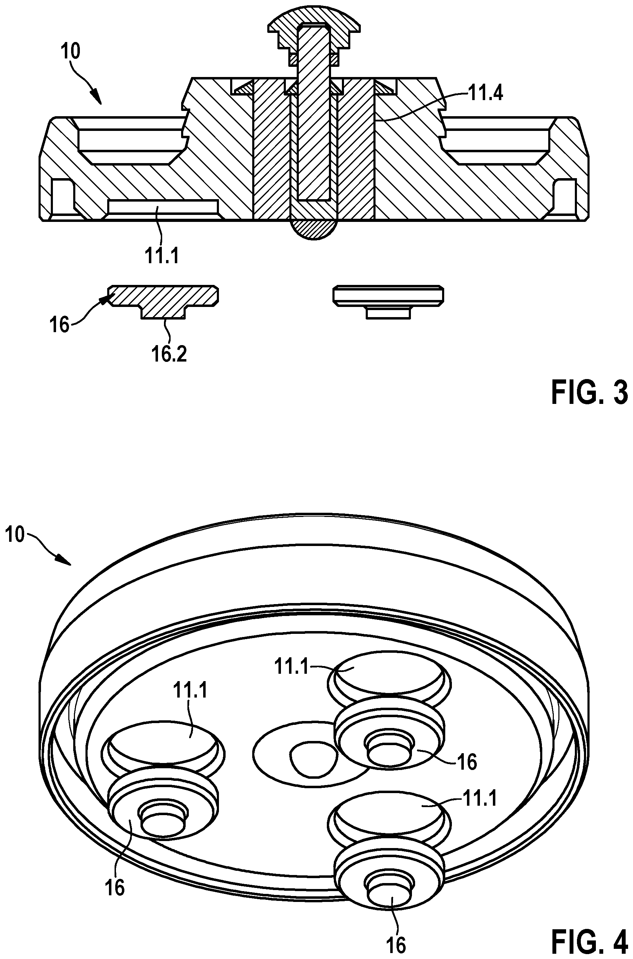

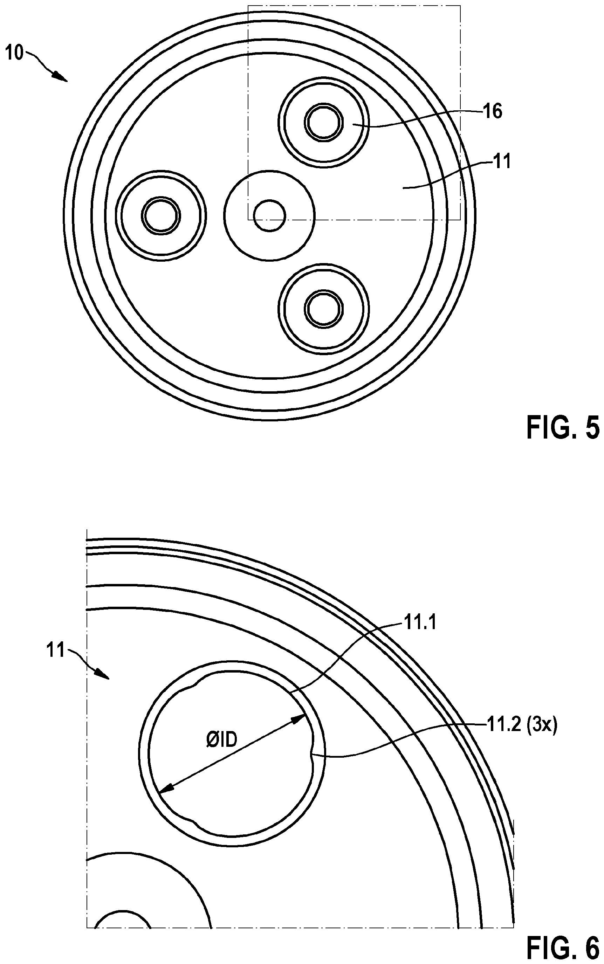

As depicted in Fig. 4 and 5, the flange 11 comprises three cylindrically shaped recesses 11.1 equally distributed and arranged on an imaginary circumferential line around the center of the flange 11 at its proximal face 11b. Each recess 11.1 is configured to receive one of three ground terminal blocks 16.

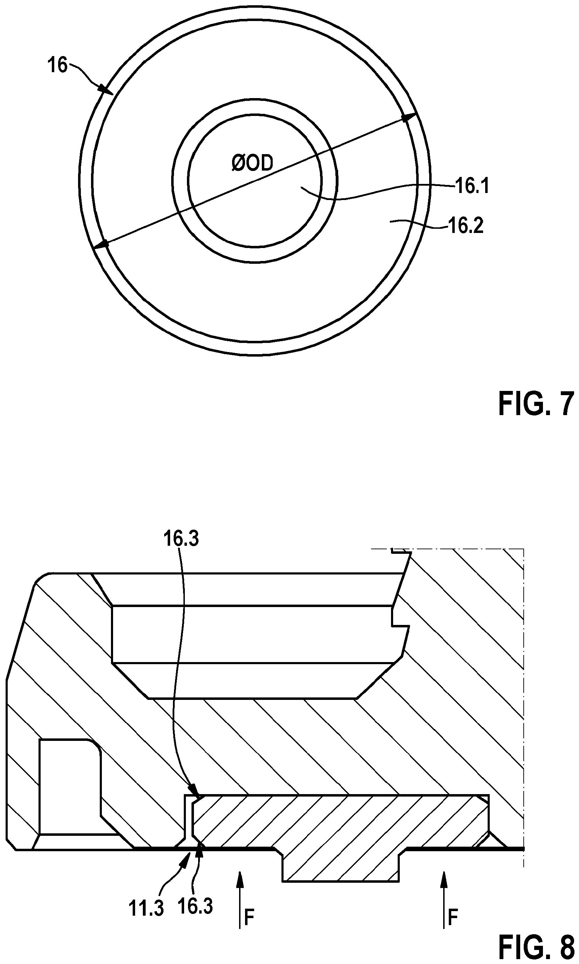

One ground terminal block 16 is shown in Fig. 7. It has a generally cylindrical shape or disc-shape, wherein there is a cylindrical projection or pin 16.1 at one of its opposite end faces. The pin 16.1 causes formation of dedicated functional surfaces with different levels at the ground terminal block 16. The protruding solder pin 16.1 at its proximal face is used for connecting the ground terminal block 16 to the electronic circuit board 30. The lower ringshaped section 16.2 forms a dedicated press-force application surface being adapted to receive the perpendicular force component F as described below. In that manner, the solder surface is not damaged during the fixation operation. The at least one electrically conducting ground terminal block 16 comprises solderable material, for example a Copper alloy or a Cu/Fe/Al alloy, and is fixed and electrically connected at its solder pin 16.1 to a respective terminal of the electrical circuitry 30 by soldering by surface mount technology (SMT) as shown in Fig. 1. In an alternative embodiment they are solder bumped, e.g. pre-tinned, at their proximal faces and/or their respective projection 16.1. Fig. 4 and 5 depict the flange 11 having the ground terminal blocks 16 prior and after each one is press-fitted or positive-locked fixed within its respective recess 11.1.

Referring to the manufacturing process of the feedthrough assembly 10 and the medical device, in a first step, a flange 11 with the feedthrough pin and the electrode tip 15 is provided, wherein the feedthrough pin 14 is fixed within the through-hole 11.4 and the electrode tip 15 is attached to the feedthrough pin 14 as described above. Then, the ground terminal blocks 16 are fixed in the corresponding recesses 11.1 of the flange 11. By applying a force F with at least a perpendicular component with regard to the proximal face of the ground terminal block 16, each ground terminal block 16 is pressed into one respective recess and is thereby friction-locking fixed within this recess 11.1, thereby establishing an electrical connection between the flange 11 and the ground terminal block 16, as well. The three ground terminal blocks 16 may be pressed into the recesses 11.1 at the same time or consecutively. After fixation, each ground terminal block 16 is in direct contact with its one end face and its side or shell face to the ground face of the respective recess 11.1 and the inner side face of the recess 11.1, respectively. The direction of the pressing force is denoted in Fig. 8 by the arrows F, wherein the pressing force is perpendicular to the proximal face 11b of the flange 11 or parallel to the axis of the respective cylindrical recess 11.1.

The lower rim of the recess 11.1 comprises a chamfer 11.3 (see Fig. 8) in order to ease introduction of one ground terminal block 16 into the recess 11.1 during manufacturing. Similarly, at both rims of shell face of the ground terminal block 16 a chamfer 16.3 is provided, as well (see Fig. 8).

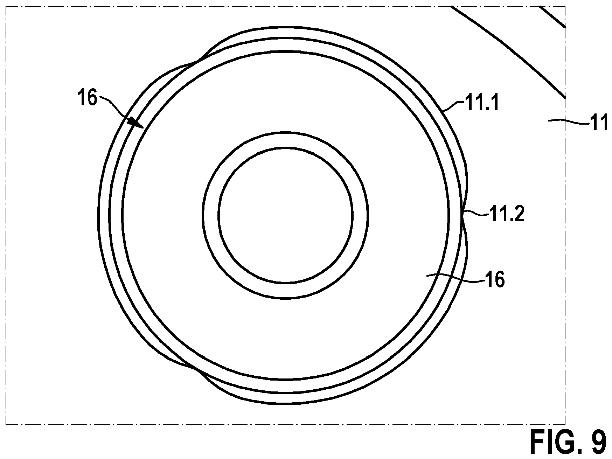

In order to provide reliable fixation of one ground terminal block 16 within one recess 11.1 the outer diameter OD of the ground terminal block 16 is slightly greater than the inner diameter ID of the recess 11.1 (see Fig. 6 and 7), so that the creation of an interference fit as shown in Fig. 9 guarantees that the ground terminal block 16 will be permanently secured inside the respective recess 11.1 of the flange 11. The overlap needed for this interference fit can be adjusted, depending on the hardness of the material of the ground terminal block 16 and the flange 11. The diameter difference of OD and ID is, for example, between 1% and 5%. Additionally, the lower rim of the recess 11.1 at which the recess merges with the proximal face 11b of the flange 11, each recess 11.1 comprises three protrusions 11.2 (see Fig. 9) protruding from the rim into the recess 11.1. The protrusions 11.2 are adapted to improve the friction-locking and/or positive-locking fixation of the respective ground terminal block 16 by sectionally narrowing the inner diameter ID of the respective recess 11.1.

The flange 11 material has a great hardness and consists of biocompatible material, e.g. Titanium or a Titanium alloy. The solderable ground terminal block 16 comprises the above mentioned material that is softer than the material of the flange 11 allowing the block 16 to deform during the fixation process and thus be permanently electrically and mechanically fixed inside the flange 11.

In another embodiment, the block 16 may be screwed into the recess 11.1 along a respective thread or fixed using a bayonet connection, whereby in these cases a rotatory force is applied along with the force component F running perpendicular to the proximal face 11b of the flange 11.

As depicted in Fig. 9, the housing 20 contains in its inner volume 22 the electronic circuit board 30. The electronic circuit board 30 may comprise, for example, a processor 30.2 and a transformer 30.4 transforming voltage signals. All components of the electronic circuit board 30 are connected to a battery 30.3 for power supply. It may further comprise a communication component for such as an antenna 30.1. These components are electrically connected to the electrode pin 14 with its electrode tip 15 to provide the electrical stimulation of the heart or processing of electrical signals determined from the heart. A ground connection of the electronic circuit board 30 for all of its components is provided by the at least one ground terminal block 16 which is electrically connecting the electronic circuit board 30 and the flange 11 and via the flange 11 to the housing 20.

The implantable medical device is assembled using the feedthrough assembly 10 manufactured as indicated above. Then, each of the ground terminal blocks 16 of such feedthrough assembly 10 is fixed and electrically connected to a respective terminal of the electronic circuit board 30 by soldering using SMT. In a next step, the electronic circuit board 30 is inserted into the housing 20. The feedthrough assembly 10 is then welded to the housing 20 to ensure that the housing 20 is hermetically closed, wherein the distal end 21 of the housing 20 is accommodated within a respective circular recess 11.5 of the flange 11. The condition of the medical device after finishing the manufacturing is depicted in Fig. 10.

## Claims

1. A feedthrough assembly (10) for an implantable medical device comprising an electrically conducting flange (11) having a distal face (11a) and a proximal face (11b), wherein the flange (11) accommodates a feedthrough pin (14) hermetically sealed within a through-hole (11.4) of the flange (11) with an electrode tip (15) located at the distal end of the feedthrough pin (14), wherein the feedthrough pin (14) is electrically insulated with regard to the flange (11), wherein the flange (11) comprises at least one electrically conducting ground terminal block (16) comprising solderable material, wherein each of the at least one ground terminal block (16) is friction-locking and/or positive-locking fixed to the flange (11) at its proximal face (11b), thereby establishing an electrical connection between the flange (11) and the at least one ground terminal block (16).

2. The feedthrough assembly according to claim 1, wherein the each of the at least one ground terminal blocks (16) is friction-locking and/or positive-locking fixed within one of at least one recesses (11.1) located at the proximal face (11b) of the flange (11).

3. The feedthrough assembly according to any one of the previous claims, wherein the at least one ground terminal block (16) comprises a material that is softer than the material of the flange (11).

4. The feedthrough assembly according to any one of the claims 2 to 3, wherein each of the at least one recesses (11.1) comprises at least one protrusion (11.2) at an inner rim of the recess (11.1) adapted to provide the friction-locking and/or positive-locking fixation of the respective ground terminal block (16).

5. The feedthrough assembly according to any one of the previous claims, wherein the at least one ground terminal block (16) is cylindrical or disc-shaped and optionally comprises a protruding solder pin (16.1) at its proximal face which comprises solderable material.

6. The feedthrough assembly according to any one of the claims 2 to 5, wherein the at least one recess (11.1) comprises a chamfer (11.3) at the proximal end of its inner side surface.

7. The feedthrough assembly according to any one of the previous claims, wherein the at least one ground terminal block (16) is solder bumped at its proximal face, preferably pre-tinned.

8. The feedthrough assembly according to any one of the previous claims, wherein at least one ground terminal block (16) comprises a chamfer (16.3) at the outer rim of its distal face.

9. An implantable medical device comprising a feedthrough assembly (10) according to any one of the previous claims and a housing (20), wherein the feedthrough assembly 10) is fixed to the housing (20) such that the housing (20) is hermetically sealed by the feedthrough assembly (10), wherein the at least one ground terminal block (16) is fixed and electrically connected to a respective terminal of an electrical circuitry (30) by soldering, wherein the electrical circuitry (30) is located within the housing (20).

10. A manufacturing method for a feedthrough assembly (10) according to any one of the claims 1 to 8, comprising the following steps:
• Providing the flange (11),
• Fixing the feedthrough pin (14) hermetically sealed and electrically insulated with regard to the flange (11) within the though-hole (11.4) of the flange (11),
• Optional attachment of the electrode tip (15) to the distal end of the feedthrough pin (14),
• Friction-locking and/or positive-locking fixing of at least one ground terminal block (16) at the proximal face (11b) of the flange (11), thereby establishing an electrical connection between the flange (11) and the at least one ground terminal block (16).

11. The manufacturing method according to claim 10, wherein the at least one ground terminal block (16) is press-fitted into or locked in place using a bayonet lock at the corresponding recess (11.1) at the proximal face of the flange (11).

12. The manufacturing method of any one of the claims 10 to 11, wherein the at least one ground terminal block (16) is solder-bumped, preferably pre-tinned, prior fixing at the flange (11).

13. A manufacturing method for an implantable medical device according to claim 9, comprising the following steps:
• Manufacturing the feedthrough assembly (10) according to any one of the claims 10 to 12,
• Fixing and electrically connecting the at least one ground terminal block (16) to a respective terminal of an electrical circuitry (30) by soldering,
• Inserting the electrical circuitry (30) within the housing (20) and afterwards welding the feedthrough assembly (10) to the implant housing (20), thereby closing the housing (20) hermetically.
